# EUROPEAN PATENT APPLICATION

(11) **EP 4 303 220 A1**
(43) Date of publication of application: **10.01.2024**
(21) Application number: 22774247.5
(22) Date of filing: 22.03.2022
(51) Int. Cl.: C07D 471/04, A61K 31/4353, A61P 35/00, A61P 37/02

(54) **CRYSTAL FORM OF FLUORINE-SUBSTITUTED PYRIDOPYRAZOLE COMPOUND AND PREPARATION METHOD THEREFOR**

(30) Priority: 23.03.2021 CN 202110309088
(71) Applicant: Jumbo Drug Bank Co., Ltd., High-tech Zone Chengdu Sichuan 610000 (CN)
(72) Inventor: ZHU, Yuchuan, Shanghai 200131 (CN); WEI, Xiawei, Chengdu, Sichuan 610000 (CN); WANG, Haobin, Shanghai 200131 (CN); SHEN, Chunli, Shanghai 200131 (CN); JIANG, Ning, Chengdu, Sichuan 610000 (CN); WU, Chengde, Shanghai 200131 (CN)
(74) Representative: Berggren Oy
(86) International application number: PCT/CN2022/082353
(87) International publication number: WO 2022/199591

(57) **Abstract**

A crystal form of a fluorine-substituted pyridopyrazole compound and a preparation method thereof. Further provided is an application of the compound and the crystal form thereof in preparation of a drug for treating a related disease.

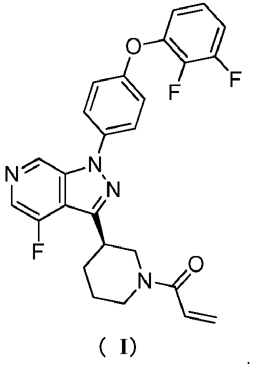

## Description

### This application claims priority to

CN202110309088.5, application date: March 23, 2021.

### Technical Field

The disclosure relates to a crystal form of a fluorine-substituted pyridopyrazole compound and a preparation method thereof, and further includes an application of the compound and the crystal form thereof in preparation of a drug for treating a related disease.

### Background

Bruton's tyrosine kinase (BTK) is a key kinase in the B cell antigen receptor (BCR) signaling pathway, BTK irreversible inhibitors bind to the active site Cys-481 of the kinase by covalent bonds, the BTK activity is inhibited, and thereby the B cell overproliferation is effectively inhibited, to achieve anti-tumor or anti-inflammatory effects.

In drugs already marketed at present, ibrutinib is an irreversible BTK inhibitor jointly developed by Pharmacyclis and Johnson&Johnson, and is approved by the Food and Drug Administration (FDA) for the treatment of mantle cell lymphoma, chronic lymphocytic leukemia, Fahrenheit macroglobulinemia, chronic graft versus host disease and the like. However, ibrutinib also has a strong inhibitory effect on other kinases besides BTK, especially on the kinases such as epidermal growth factor receptor (EGFR), interleukin-2-inducible T-cell kinase (ITK), and tyrosine kinase expressed in hepatocelluar carcinoma (TEC), which may lead to more serious adverse reactions such as rash, diarrhea, and hemorrhage. Therefore, it is necessary to develop a new type of highly active and highly selective BTK inhibitors for the treatment of related diseases in this field.

### Summary

The disclosure provides a crystal form A of a compound in Formula (I), wherein, its X-ray powder diffractometer (XRPD) spectrum has characteristic diffraction peaks at the following 2θ angles: 17.7805±0.2000°, 22.0193±0.2000°, 27.4192±0.2000°,

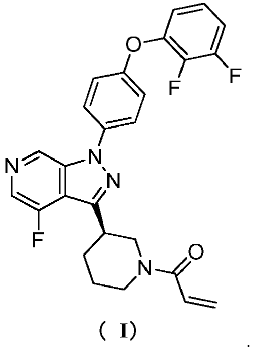

In some schemes of the disclosure, the XRPD spectrum of the above crystal form A has characteristic diffraction peaks at the following 2θ angles: 15.0010±0.2000°, 17.0603±0.2000°, 17.7805±0.2000°, 22.0193±0.2000°, 23.5013±0.2000°, 27.4192±0.2000°.

In some schemes of the disclosure, the XRPD spectrum of the above crystal form A has characteristic diffraction peaks at the following 2θ angles: 8.1387±0.2000°, 15.0010±0.2000°, 16.1591±0.2000°, 17.0603±0.2000°, 17.7805±0.2000°, 22.0193±0.2000°, 23.5013±0.2000°, 27.4192±0.2000°.

In some schemes of the disclosure, the XRPD spectrum of the above crystal form A has characteristic diffraction peaks at the following 2θ angles: 8.1387±0.2000°, 13.6809±0.2000°, 15.0010±0.2000°, 16.1591±0.2000°, 17.0603±0.2000°, 17.7805±0.2000°, 22.0193±0.2000°, 23.5013±0.2000°, 24.0218±0.2000°, 27.4192±0.2000°.

In some schemes of the disclosure, the XRPD spectrum of the above crystal form A has characteristic diffraction peaks at the following 2θ angles: 17.7805±0.2000°, 22.0193±0.2000°, and/or 27.4192±0.2000°, and/or 8.1387±0.2000°, and/or 9.8194±0.2000°, and/or 13.6809±0.2000°, and/or 15.0010±0.2000°, and/or 16.1591±0.2000°, and/or 17.0603±0.2000°, and/or 18.4386±0.2000°, and/or 19.5400±0.2000°, and/or 21.3193±0.2000°, and/or 23.1597±0.2000°, and/or 23.5013±0.2000°, and/or 24.0218±0.2000°, and/or 26.1791±0.2000°, and/or 26.6006±0.2000°, and/or 27.1199±0.2000°, and/or 29.1595±0.2000°, and/or 29.7190±0.2000°, and/or 30.8417±0.2000°, and/or 31.2196±0.2000°, and/or 32.2992±0.2000°, and/or 32.9612±0.2000°, and/or 33.7773±0.2000°, and/or 34.3779±0.2000°, and/or 35.1796±0.2000°, and/or 37.1408±0.2000°.

In some schemes of the disclosure, the XRPD spectrum of the above crystal form A has characteristic diffraction peaks at the following 2θ angles: 8.1387°, 9.8194°, 13.6809°, 15.0010°, 16.1591°, 17.0603°, 17.7805°, 18.4386°, 19.5400°, 21.3193°, 22.0193°, 23.1597°, 23.5013°, 24.0218°, 26.1791°, 26.6006°, 27.1199°, 27.4192°, 29.1595°, 29.7190°, 30.8417°, 31.2196°, 32.2992°, 32.9612°, 33.7773°, 34.3779°, 35.1796°, 37.1408°.

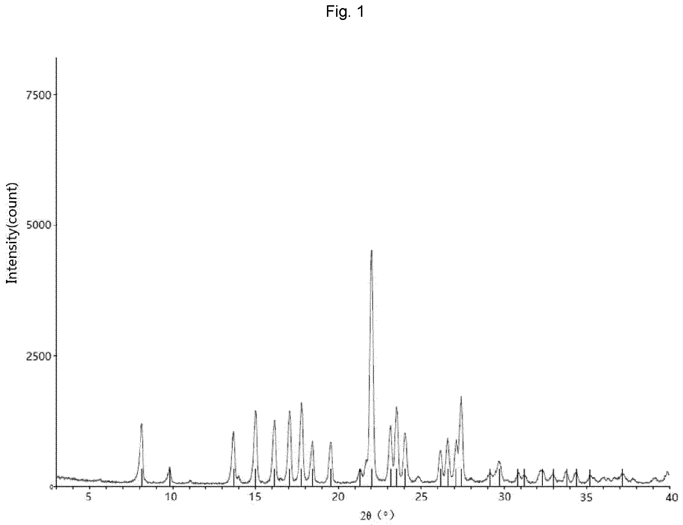

In some schemes of the disclosure, the XRPD spectrum of the above crystal form A is basically shown in Fig. 1.

In some schemes of the disclosure, XRPD spectrum analysis data of the above crystal form A is shown in Table 1:

**Table 1: XRPD spectrum analysis data of crystal form A of compound in Formula (I)**

| Numb er | 2θ angle (°) | Interplan ar distance (A) | Intensi ty (count ) | Relati ve intensi ty (%) | Numb er | 2θ angle (°) | Interplan ar distance (A) | Intensi ty (count ) | Relati ve intensi ty (%) |
|---|---|---|---|---|---|---|---|---|---|
| 1 | 8.138 7 | 10.8548 | 1132 | 25.5 | 15 | 26.17 91 | 3.4013 | 615 | 13.8 |
| 2 | 9.819 4 | 9.0002 | 307 | 6.9 | 16 | 26.60 06 | 3.3483 | 841 | 18.9 |
| 3 | 13.68 09 | 6.4673 | 990 | 22.3 | 17 | 27.119 9 | 3.2854 | 806 | 18.1 |
| 4 | 15.00 10 | 5.9011 | 1385 | 31.2 | 18 | 27.41 92 | 3.2502 | 1635 | 36.8 |
| 5 | 16.15 91 | 5.4807 | 1198 | 26.9 | 19 | 29.15 95 | 3.0600 | 160 | 3.6 |
| 6 | 17.06 03 | 5.1931 | 1382 | 31.1 | 20 | 29.71 90 | 3.0037 | 388 | 8.7 |
| 7 | 17.78 05 | 4.9844 | 1529 | 34.4 | 21 | 30.84 17 | 2.8969 | 212 | 4.8 |
| 8 | 18.43 86 | 4.8079 | 800 | 18.0 | 22 | 31.21 96 | 2.8627 | 137 | 3.1 |
| 9 | 19.54 00 | 4.5393 | 777 | 17.5 | 23 | 32.29 92 | 2.7694 | 248 | 5.6 |
| 10 | 21.31 93 | 4.1643 | 265 | 6.0 | 24 | 32.96 12 | 2.7153 | 201 | 4.5 |
| 11 | 22.0193 | 4.0335 | 4446 | 100.0 | 25 | 33.7773 | 2.6515 | 234 | 5.3 |
| 12 | 23.15 97 | 3.8374 | 1081 | 24.3 | 26 | 34.37 79 | 2.6065 | 202 | 4.5 |
| 13 | 23.50 13 | 3.7824 | 1445 | 32.5 | 27 | 35.17 96 | 2.5490 | 135 | 3.0 |
| 14 | 24.02 18 | 3.7016 | 930 | 20.9 | 28 | 37.14 08 | 2.4187 | 163 | 3.7 |

In some schemes of the disclosure, a differential scanning calorimeter (DSC) curve of the above crystal form A has a starting point of an endothermic peak at 107.89±3.00 °C, and a peak of an exothermic peak at 147.76±3.00°C.

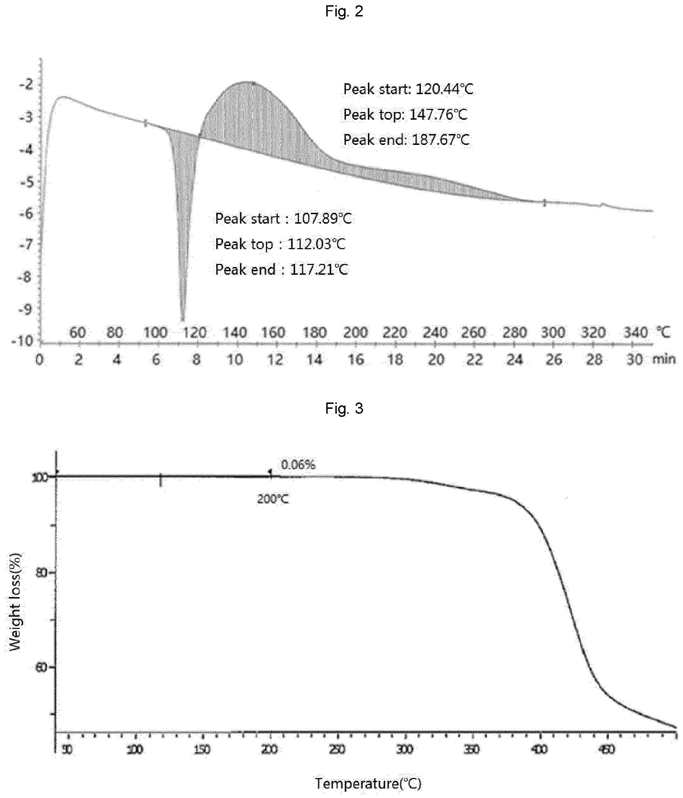

In some schemes of the disclosure, a DSC spectrum of the above crystal form A is shown in Fig. 2.

In some schemes of the disclosure, the weight loss of a thermal gravimetric analyzer (TGA) curve of the above crystal form A reaches 0.06% at 200.0±3 °C.

In some schemes of the disclosure, a TGA spectrum of the above crystal form A is shown in Fig. 3.

In some schemes of the disclosure, single crystal X-ray diffraction data of the above crystal form A are as follows: monoclinic crystal system, space group C2, crystal cell parameters *a*=22.0128(6) Ǻ , b=12.7542(3) Ǻ , *c*=16.0152(4) Ǻ , α = γ = 90°, β = 90.4900(10)°, volume V=4496.2(2) Ǻ³, absolute configuration parameter Flack value of 0.04(3).

The disclosure further provides a preparation method for the crystal form A of the compound in Formula (I), including:
1) The compound in Formula (I) is added to a solvent to form solution;
2) An anti-solvent is added to the solution, it is stirred at a certain temperature for a period of time, and filtered, a filter cake is dried under reduced pressure.

Wherein, the solvent is an ester solvent, an ether solvent, or an alcohol solvent.

The anti-solvent is n-heptane, n-hexane, or water.

The stirring temperature is 0-40°C.

The stirring time is 12-48 hours.

The disclosure further provides a preparation method for the crystal form A of the compound in Formula (I), including:
1) The compound in Formula (I) is added to a solvent to form solution;
2) An anti-solvent is added to the solution, it is stirred at 30 °C, and filtered, a filter cake is dried under a reduced pressure.

Wherein, the solvent is ethyl acetate, tetrahydrofuran, or ethanol.

The anti-solvent is n-heptane, n-hexane, or water.

The stirring time is 12-20 hours.

The disclosure further provides an application of the above crystal form A in preparation of a drug for treating a disease related to BTK.

In some schemes of the disclosure, the disease related to BTK is hematoma or autoimmune disease.

### Definition and Description

Unless otherwise specified, the following terms and phrases used in this article are intended to have the following meanings. A specific phrase or term should not be considered uncertain or unclear without a specific definition, but should be understood according to the ordinary meaning. When a product name appears in this article, it is intended to refer to its corresponding product or its active ingredient.

Intermediate compounds of the disclosure may be prepared by various synthesis methods well-known to those skilled in the art, including specific implementation modes listed below, implementation modes formed by its combination with other chemical synthesis methods, and equivalent replacement modes well-known to those skilled in the art. Preferred implementation modes include, but are not limited to, embodiments of the disclosure.

Chemical reactions of the specific implementation modes of the disclosure are completed in suitable solvents, and the solvents must be suitable for the chemical changes of the disclosure and required reagents and materials thereof. In order to obtain the compound of the disclosure, it is sometimes necessary for those skilled in the art to modify or select the synthesis steps or reaction processes based on the existing implementation modes.

The structure of the compound of the disclosure may be confirmed by conventional methods well-known to those skilled in the art. If the disclosure involves the absolute configuration of the compound, the absolute configuration may be confirmed by conventional technical means in the art. For example, single crystal X-ray diffraction (SXRD) is used to collect diffraction intensity data from a cultured single crystal by using a Bruker D8 venture diffractometer, a light source is CuKα radiation, and scanning mode: ϕ/ω scanning. After relevant data is collected, a direct method (Shelxs97) is further used to analyze the crystal structure, so that the absolute configuration may be confirmed.

The disclosure may be described in detail below by the embodiments, and these embodiments do not imply any limitations to the disclosure.

All solvents used in the disclosure are commercially available and may be used without further purification.

The solvents used in the disclosure may be commercially available.

Compounds are named according to conventional naming principles in this field or by using software, and commercially available compounds are named by using supplier catalog names.

### Technical effect

The compound of the disclosure is stable in crystal form, has slight hygroscopicity, and is less affected by photothermal effects. The compound of the disclosure, as a highly active and highly selective BTK kinase inhibitor, shows the good selectivity for EGFR, ITK, and TEC kinases.

### X-ray powder diffractometer (XRPD) method of the disclosure

Instrument name: X-ray diffractometer.

Instrument model: DX-2700BH.

Instrument manufacturer: Dandong Haoyuan Instrument Co., Ltd.

Method parameter:
Light tube: Cu, k-Alpha (λ=1.54059Å).
Light tube voltage: 40 kV, and light tube current: 40 mA.
Divergence slit: 0.3 mm.
Detector slit: 1 mm.
Anti-scattering slit: 1 mm.
Scanning range: 3-40 deg.
Step diameter: 0.02 deg.
Step length: 0.5 seconds.

Test method: a sample is placed on a sample board, to guarantee that the surface of the sample board is flat. Finally, the sample board is placed in the X-ray diffractometer for testing.

### Differential scanning calorimeter (DSC) method of the disclosure

Instrument model: DSC1 differential scanning calorimeter.

Test method: a sample (2.97 mg) is taken and placed in a DSC high-pressure crucible, after being sealed by a pressing sheet, it is tested, and the sample is heated from 40°C to 350°C at a heating rate of 10°C/min.

### Thermal gravimetric analyzer (TGA) method of the disclosure

Instrument model: Mettler TGA2SF/1100.

Test method: a sample (2-5 mg) is taken and placed in a TGA alumina crucible for testing. Under the condition of 20 mL/min N₂, the sample is heated from 40°C to 500°C at a heating rate of 10 °C/min, and the weight loss from 40°C to 200°C is 0.06%.

### Dynamic vapor sorption (DVS) method of the disclosure

Instrument model: SMS DVS Intrinsic Plus dynamic steam adsorption instrument.

Test condition: a sample (10-20 mg) is taken and placed in a DVS sample tray for testing.

Detailed DVS parameters are as follows:

Temperature: 25°C.

Balance: dm/dt=0.002%/min (minimum: 10 min, and maximum: 180 min).

Drying: it is dried at 0% relative humidity (RH) until dm/dt≤0.002%/min or maximum 180 min.

RH (%) test step: 10% (0%-90%), 5% (90-95%).

RH (%) test step range: 0%-95%-0%.

**Table 2: Classification of hygroscopicity evaluation**

| Hygroscopic classification | ΔW% |
|---|---|
| Deliquesce | Absorb sufficient moisture to form liquid |
| Extremely hygroscopic | ΔW%≥15% |
| Hygroscopic | 15%>ΔW%≥2% |
| Slightly hygroscopic | 2%>ΔW%≥0.2% |
| No or almost no hygroscopicity | ΔW%<0.2% |

| | |
|---|---|
| Note: ΔW% represents a moisture absorption gain of a test substance at 25±1 °C and 80±2% RH. | |

### Brief Description of the Drawings

Fig. 1 is an XRPD spectrum of Cu-Kα radiation of a crystal form A of a compound in Formula (I).
Fig. 2 is a DSC spectrum of the crystal form A of the compound in Formula (I).
Fig. 3 is a TGA spectrum of the crystal form A of the compound in Formula (I).
Fig. 4 is a DVS spectrum of the crystal form A of the compound in Formula (I).

### Detailed Description of the Embodiments

In order to understand the content of the disclosure better, it is further described below in combination with specific embodiments, but the specific implementation modes are not intended to limit the content of the disclosure.

### Embodiment 1: Preparation of compound in Formula (I)

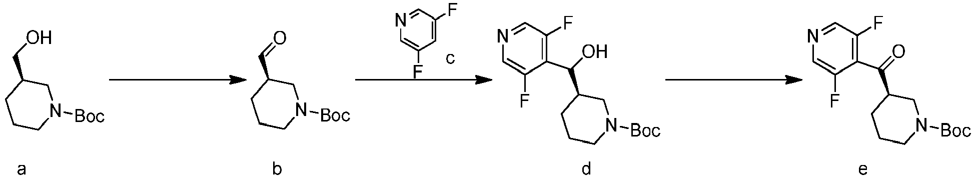

### Step 1: Synthesis of compound b

Preparation of sodium hypochlorite solution: NaHCOs (11.00 g, 130.94 mmol, and 7.05 e-2 eq) was dissolved in NaClO (2.42 kg, 2.60 mol, 2 L, 8% purity, and 1.40 eq). KBr (2 M, 96.00 mL, and 1.03 e-1 eq) (aqueous solution) was added to dichloromethane (1.6 L) solution of a compound a (400 g, 1.86 mol, and 1 eq) and 2,2,2,6,6-tetramethylpiperidine oxide (2.98 g, 18.97 mmol, 1.02 e-2 eq), it was maintained at 5-35 °C in an ice bath, and the above sodium hypochlorite solution was dropwise added. After being added dropwise, it was maintained at 25-35 °C and reacted continuously for 30 min. After reaction solution was placed stilly, the solution was separated, an aqueous phase obtained by separation was extracted with dichloromethane (1.6 L), an organic phase combined was washed with 1 M hydrochloric acid (containing KI (616.85 g, 3.72 mol, and 2 eq), 1.6 L) and 10% sodium thiosulfate solution (1.6 L) sequentially, and the organic phase was dried with anhydrous sodium sulfate, and filtered. A filtrate was concentrated under a reduced pressure to dry. A compound b was obtained. LCMS (ESI): m/z (M-55)⁺: 158.7.

### Step 2: Synthesis of compound d

Under the protection of dry ice ethanol bath and nitrogen, n-butyl lithium (2.5 M, 1.13 L, and 1.07 eq) was dropwise added to tetrahydrofuran solution (2.1 L) of a compound c (309.65 g, 2.69 mol, and 1.02 eq), and it was maintained at -68 to -40°C during dropwise addition. After being added dropwise, it was reacted at -68 to -40°C for 1 hr. Tetrahydrofuran (0.7 L) solution of the compound b (563 g, 2.64 mol, and 1 eq) was added dropwise, and it was maintained at -68 to -40 °C during dropwise addition. After being added dropwise, the temperature was slowly raised to 0°C and it was reacted for 2 hr. Under stirring, saturated ammonium chloride solution (1.2 L) was added to reaction solution, and the reaction solution was concentrated to about 3 L under a reduced pressure and extracted with ethyl acetate (1.2 L*3). An organic phase combined was washed with 1 M hydrochloric acid (1.2 L) and semi-saturated salt water (1.2 L) sequentially, and the organic phase was dried with anhydrous sodium sulfate, and filtered. A filtrate was concentrated under a reduced pressure to dry. After being concentrated, a compound d was obtained. LCMS (ESI): m/z 272.9.

### Step 3: Synthesis of compound e

KBr (2 M, 74.00 mL, and 1.05 e-1 eq) (aqueous solution) was added to dichloromethane (1.8 L) solution of the compound d (464 g, 1.41 mol, and 1 eq) and 2,2,2,6,6-tetramethylpiperidine oxide (2.23 g, 14.16 mmol, and 0.01 eq), it was maintained at 10-15°C in an ice bath, NaClO (1.84 kg, 1.98 mol, 1.72 L, 8% purity, and 1.4 eq) was dropwise added and it was maintained at 10-15 °C and reacted for 10 min. NaHCOs (40 g, 476.15 mmol, and 3.37 e-1 eq) was supplemented, and it was reacted continuously for 30 min. Reaction solution was stilly placed and separated, and an aqueous phase obtained by separation was extracted with dichloromethane (9 L). An organic phase combined was washed with 1 M hydrochloric acid (containing 2.35 kg of KI, 2 eq, and 9 L) and 10% sodium thiosulfate solution (9 L) sequentially, and the organic phase was dried with anhydrous sodium sulfate, and filtered. A filtrate was concentrated under a reduced pressure to dry. After being detected by a supercritical fluid chromatography (chromatographic column: Chiralpak AD-3 150×4.6 mml.D, 3 µm; mobile phase: A: supercritical carbon dioxide, B: 0.05% ethanol solution of diethylamine; gradient: B was increased from 5% to 40% within 5.5 min, and it was maintained at 5% for 1.2 min; flow rate: 2.5 mL/min; column temperature: 40 °C; and wavelength: 220 nm), it was analyzed as a single configuration compound. A compound e was obtained. LCMS (ESI): m/z 271.1.

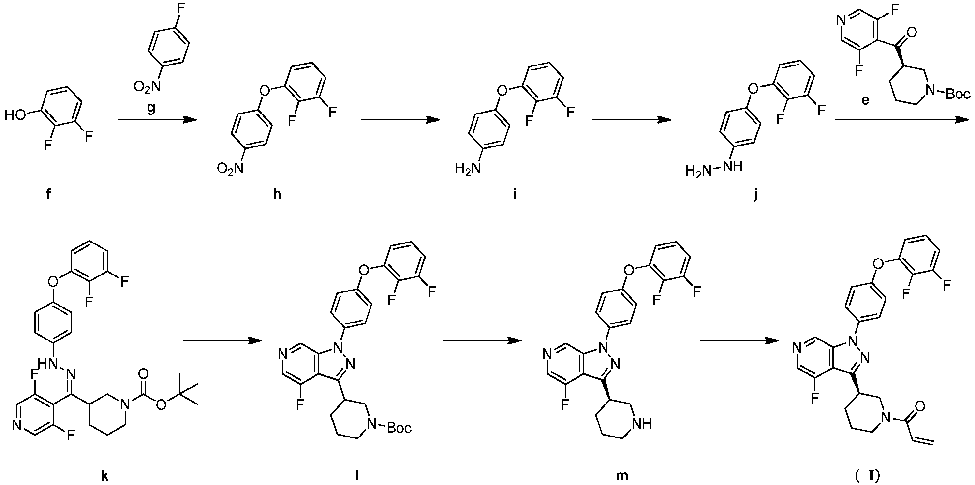

### Step 4: Synthesis of compound h

Under stirring, K₂CO₃ (1.47 kg, 10.63 mol, and 1.5 eq) was added to acetonitrile (5 L) solution of a compound g (1 kg, 7.09 mol, 751.88 mL, and 1 eq) and a compound f (1.11 kg, 8.50 mol, and 1.2 eq), the temperature was raised to 75 °C and it was reacted for 13 hr. The reaction solution was cooled to room temperature, a supernatant was added to 50 L of water after being placed stilly, and after 10 min, a large number of solids were generated, it was stirred continuously for 10 min; and the remaining reaction solution was filtered, a filter cake was washed with acetonitrile (1 L), a filtrate combined was added to 10 L of water under stirring, and after 10 min, a large number of solids were generated, it was stirred continuously for 10 min. Two batches of the above suspended solids were combined and filtered, a filter cake was washed with water (1 L), it was suction-filtered continuously to dry, and the filter cake was collected. A compound h was obtained. ¹H NMR(400MHz,CDCl₃)δ8.20-8.23(m,2H), 7.11-7.14(m,2H), 7.02-7.04(m,2H), 6.96-7.01(m,1H).

### Step 5: Synthesis of compound i

Pd/C (4 g, 10% purity) (wet palladium carbon) was added to methanol (1 L) solution of the compound h (200 g, 796.22 mmol, and 1 eq) in a hydrogenation flask, it was replaced with argon for three times, replaced with hydrogen for three times, and reacted at room temperature (25°C) for 16 hr under a hydrogen pressure (30 psi). Reaction solution (spread diatomaceous earth) was filtered, a filter cake was washed with methanol (1 L), and a filtrate combined was concentrated under a reduced pressure to dry. A compound i was obtained. LCMS (ESI): m/z (M+1)⁺: 222.0.

### Step 6: Synthesis of compound j

In an ice water bath (5°C), the compound i (350 g, 1.58 mol, and 1 eq) was added to concentrated hydrochloric acid (2 L) and water (1 L), and solution of water (0.5 L) dissolved with NaNO₂ (218.35 g, 3.16 mol, and 2 eq) was dropwise added to suspension obtained. The internal temperature was maintained at 5-15 °C during dropwise addition, and it was dropwise added for ~0.5 hr. After being added dropwise, it was maintained at 5-10 °C and reacted for 1 hr. Solution of concentrated hydrochloric acid (1 L) dissolved with SnCl₂·2H₂O (1.43 kg, 6.33 mol, and 4.00 eq) was dropwise added to reaction solution. The internal temperature was maintained at 5-15 °C during dropwise addition, and it was dropwise added for about 3 hr total. After being added dropwise, water (0.75 L) was supplemented so that solids were uniformly distributed in a reaction system. The temperature was slowly raised to room temperature (25 °C) and it was reacted continuously for 4 hr. Reaction solution was filtered, a filter cake was washed with water (3 L), and the filter cake was collected. The filter cake was divided into 4 parts, each of which was dispersed in mixed solution of methanol (0.5 L) and dichloromethane (4 L). PH was adjusted to about 14 by using 6 M NaOH solution, and the solution was separated after being placed stilly. An aqueous phase obtained was extracted with dichloromethane (2 L*2), and an organic phase was collected; and the organic phase was combined and filtered, a filter cake was washed with 500 mL of dichloromethane, a filtrate combined was separated, an aqueous phase was extracted with dichloromethane (1 L*2), and an organic phase was collected. The organic phase combined was dried with anhydrous sodium sulfate, and filtered, and a filtrate was concentrated under a reduced pressure to dry. A compound j was obtained. LCMS (ESI): m/z (M+1)⁺: 237.1.

### Step 7: Synthesis of compound k

AcOH (15.21 mol, 870.00 mL, and 4.96 eq) was added to ethanol (5 L) solution of a compound e (1 kg, 3.06 mol, and 1 eq) and the compound j (1 kg, 4.23 mol, and 1.38 eq), and it was reacted at room temperature (25 °C) for 12 hr. Reaction solution was concentrated under a reduced pressure to dry. A compound k was obtained.

### Step 8: Synthesis of compound I

Cs₂CO₃ (2.8 kg, 8.59 mol, and 2.46 eq) was added to N,N-dimethylformamide (6.5 L) solution of the compound k (1.9 kg, 3.49 mol, and 1 eq), the temperature was raised to 100 °C and it was reacted for 1.5 hr. Reaction solution was filtered, and a filtrate was concentrated by using an oil pump under a reduced pressure to dry; and a filter cake was washed with dichloromethane (1 L), and a filtrate combined was concentrated to dry under a reduced pressure by a water pump and oil pump sequentially. A concentrate was dispersed in ethanol (8 L), it was filtered after being stirred for 1 hr, and a filter cake was washed with 1 L of ethanol. A filtrate combined was concentrated to dry under a reduced pressure, and after being purified by a silica gel column (petroleum ether: ethyl acetate=1:0-4:1, and 10% dichloromethane was added), a concentrate obtained was dispersed in ethyl acetate (1.1 L). Under stirring, 3.3 L of petroleum ether was added, and it was stirred at room temperature (25 °C) for 3 hr. It was filtered, a filter cake was washed with 200 mL of petroleum ether, and the filter cake was collected, and concentrated to dry under a reduced pressure. A compound I was obtained. LCMS (ESI): m/z: 525.3 [M+1]. After being detected by the supercritical fluid chromatography (chromatographic column: ChiralpakAD-3 50×4.6 mml.D, 3 µm; mobile phase: A: supercritical carbon dioxide, B: 0.05% ethanol solution of diethylamine; gradient: B was increased from 5% to 40% within 5 min, to 5% within 0.5 min, and it was maintained at 5% for 1.5 min; flow rate: 2.5 mL/min; column temperature: 35 °C; and wavelength: 220 nm), it was analyzed as a racemic compound.

### Step 9: Synthesis of compound m

Methanol solution (3.8 L) of HCl (4 M, 3.80 L, and 6.33 eq) was dropwise added to methanol (2.6 L) suspension dissolved with the compound I (1.26 kg, 2.40 mol, and 1 eq), and after being added dropwise, it was reacted at room temperature (25 °C) for 0.5 hr, and solution becomes clear at this time. Reaction solution was concentrated to dry under a reduced pressure. A concentrate was dissolved in mixed solution of 4 L of dichloromethane and 400 mL of methanol, pH was adjusted to 14 by using 6 M sodium hydroxide solution, it was stirred continuously for 0.5 hr, and the solution was separated after being placed stilly. An aqueous phase obtained was extracted with 3 L of dichloromethane, an organic phase combined was dried with anhydrous sodium sulfate, and filtered, and a filtrate was concentrated to dry under a reduced pressure. After being detected by the supercritical fluid chromatography (chromatographic column: Chiralpak IG-3 50×4.6 mml.D, 3 µm; mobile phase: A: supercritical carbon dioxide, B: 0.05% ethanol solution of diethylamine; gradient: B was increased from 5% to 40% within 2 min, to 5% within 0.5 min, and it was maintained at 40% for 1.2 min, and maintained at 5% for 0.8 min; flow rate: 4 mL/min; column temperature: 35 °C; and wavelength: 220 nm), it was analyzed as a racemic compound. After being separated by the supercritical fluid chromatography (SFC) (chromatographic column: DAICEL CHIRALPAK AD (250 mm*50 mm, 10 um); mobile phase: A: supercritical carbon dioxide, B: [0.1% NH₃H₂O MEOH]; B%: 50%-50%, min), it was purified to obtain m, and the retention time was 2.584 min. LCMS (ESI): m/z: 425.2 [M+1].

### Step 10: Synthesis of compound in Formula (I)

Na₂CO₃ (105 g, 990.66 mmol, and 1.5 eq) was added to tetrahydrofuran (2.8 L) and water (2.8 L) solution of the compound m (280 g, 659.73 mol, and 1 eq), tetrahydrofuran (0.7 L) solution dissolved with acryloyl chloride (65.88 g, 727.92 mmol, 59.35 mL, and 1.1 eq) was dropwise added to reaction solution, and it was reacted at room temperature (28 °C) for 0.5 hr. After tetrahydrofuran (50 mL) solution of acryloyl chloride (10.6 g, 117.12 mmol, 9.55 mL, and 1.78 e-1 eq) and Na₂CO₃ (70 g, 660.45 mmol, and 1.00 eq) were supplemented, it was continuously reacted at room temperature (28 °C) for 0.5 hr. pH of reaction solution was adjusted to 6 by using 1 N hydrochloric acid, and it was extracted with dichloromethane (3.5 L*2). An organic phase combined was dried with anhydrous sodium sulfate, and filtered, and a filtrate was concentrated to dry under a reduced pressure. A concentrate was purified by the silica gel column (petroleum ether: ethyl acetate=1:0-1:1, and 10% dichloromethane was added for solubilization). A crude product obtained was dissolved in dichloromethane (1 L), it was concentrated to dry under a reduced pressure, and this operation was repeated for three times before being transferred to an oil pump for reduced pressure concentration, to obtain the compound in Formula (I). After being detected by SFC (chromatographic column: Cellulose 2150×4.6 mml.D, 5 µm; mobile phase: A: supercritical carbon dioxide, B: 0.05% ethanol solution of diethylamine; gradient: B was increased from 5% to 40% within 5 min, and it was maintained at 40% for 2.5 min, and returned to 5% balance for 2.5 min; flow rate: 2.5 mL/min; column temperature: 35 °C; and wavelength: 220 nm), it was analyzed as a single configuration compound, and the retention time was 6.916 min. ¹HNMR(400MHz, DMSO-*d*₆)δ9.08(s, 1H), 8.32(s, 1H), 7.85(d, J=8.8Hz, 2H), 7.24-7.33(m, 4H), 7.09-7.11(m, 1H), 6.80-6.7582, 1H), 6.08(t, J=16.1 Hz, 1H), 5.59-5.70(m, 1H), 4.70-4.72(m, 0.5H), 4.32(t, J=12.7Hz, 1H), 4.07-4.10(m, 0.5H), 3.57-3.44(m, 0.5H), 3.33-3.15(m, 1.5H), 3.12-3.03(m, 0.5H), 3.03-2.88(m, 0.5H), 2.27-2.16(m, 1H), 2.05-1.78(m, 2H), 1.68-1.49(m, 1H); LCMS(ESI):m/z(M+1)⁺:479.3.

### Configuration confirmation of compound in Formula (I):

At room temperature, the compound (I) in Formula (I) (100 mg, 209.00 µmol, and 1 eq) was dissolved in dichloromethane (DCM) (0.3 mL). Under stirring, n-heptane (1.5 mL) was dropwise added, an apparent viscous substance appears, and it remains undissolved when being heated (40-50 °C). It was cooled to room temperature and stilly placed overnight, and a yellow oily substance appears. It was stilly placed continuously at room temperature in dark for seven months, and a transparent crystal appears in the system. It was taken for testing single crystal diffraction, and it was confirmed as the above configuration.

### Single crystal diffraction test:

Instrument model: Single crystal X-ray diffractometer (SC-XRD) (D8 VENTURE).

Conclusion: the molecular formula of the compound in Formula (I) was C₂₆H₂₁F₃N₄O₂, crystal system: Monoclinic, space group: C2, crystal cell parameters: *a*=22.0128(6) Ǻ, *b*=12.7542(3) Ǻ, *c*=16.0152(4) Ǻ, α=γ=90°, β=90.4900(10)°, volume: V=4496.2(2) Ǻ³, and the absolute configuration parameter Flack value was 0.04 (3).

### Embodiment 2: Preparation of crystal form A of compound in Formula (I)

At room temperature (30 °C), the compound in Formula (I) (79.3 g, 165.74 mmol, and 1 eq) was dissolved in ethyl acetate (80 mL), n-heptane (400 mL) was slowly added dropwise, and it was dropwise added until about 100 mL. A viscous solid was precipitated, the dripping speed was slowed down, and the stirring speed was increased. After about 2 hours of dropwise addition, the large solid (attached to a bottle wall) was dispersed, and it was stirred continuously at room temperature (30 °C) for 16 hours. It was filtered, and a filter cake was collected. The filter cake was dried under a reduced pressure, and the filter cake obtained was vacuum-dried at 60 °C overnight (16 h), to obtain the crystal form A of the compound in Formula (I).

Ethanol (60 mL) was added to a 500 mL single-port bottle loaded with the compound (30.00 g, 62.70 mmol, and 1 eq) in Formula (I), it was heated to 80 °C, and stirred until almost dissolved. It was cooled to room temperature (30 °C), the crystal form A (about 300 mg) of the compound in Formula (I) was added, it was stirred at room temperature (30 °C) for 2 hours, water (120 mL) was dropwise added, and it was stirred at room temperature (30 °C) for 16 hours. A large solid was dispersed, and it was stirred again for 2 hours. It was filtered, a filter cake was collected, and it was vacuum-dried at 90 °C for 32 hours, to obtain the crystal form A of the compound in Formula (I).

### Embodiment 3: Preparation of compound 1

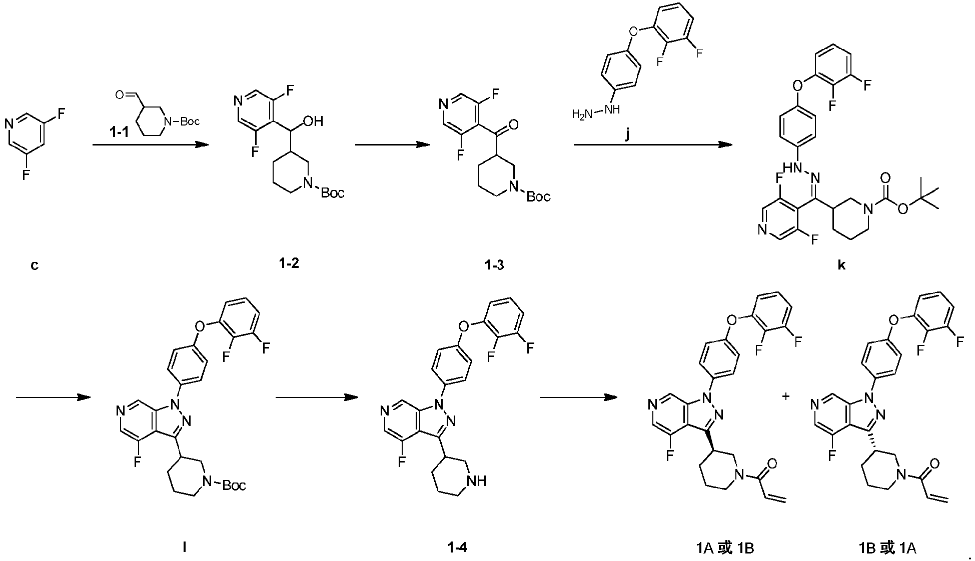

### Step 1: Preparation of compound 1-2

Preparation of lithium diisopropylamide (LDA) solution: under the nitrogen protection, n-butyl lithium (2.5 M, 7.04 mL, and 1.1 eq) was dropwise added to anhydrous tetrahydrofuran (30 mL) solution of diisopropylamine (1.70 g, 16.80 mmol, 2.37 mL, and 1.05 eq) at -78 °C, a mixture obtained was heated to 0 °C and it was reacted for 0.5 hours, then it was cooled again to -78 °C for later use.

Under the protection of nitrogen at -78 °C, the above LDA solution was dropwise added to anhydrous tetrahydrofuran (5 mL) solution dissolved with a compound c (1.84 g, 15.99 mmol, and 1 eq), and a mixture obtained was reacted at -78 °C for 1 hour. Anhydrous tetrahydrofuran (5 mL) solution dissolved with the compound 1-1 (3.41 g, 15.99 mmol, and 1 eq) was dropwise added to reaction solution, and a mixture obtained was gradually heated to room temperature (24 °C) and it was continuously reacted for 16 hours. Saturated ammonium chloride solution was added to the system, ethyl acetate (20 mL) was added, and the solution was separated and extracted. An organic phase was washed with saturated salt water (10 mL), dried with anhydrous sodium sulfate, and filtered. A filtrate was concentrated under a reduced pressure. It was separated and purified by a column chromatography, to obtain a compound 1-2. LCMS: MS (ESI) m/z 272.9.

### Step 2: Preparation of compound 1-3

At 0 °C, a Dess-Martin oxidant (7.84 g, 18.47 mmol, 5.72 mL, and 1.2 eq) was added to anhydrous dichloromethane (300 mL) solution dissolved with the compound 1-2 (5.07 g, 15.44 mmol, and 1 eq), and it was gradually raised to room temperature (26 °C) and reacted for 3 hours. Saturated sodium bicarbonate solution (200 mL) and dichloromethane (400 mL) were added to the system, and it was filtered. A filtrate was separated, an organic phase was washed with saturated salt water (100 mL), and it was dried with anhydrous sodium sulfate, and filtered. A filtrate was concentrated under a reduced pressure. A compound 1-3 was obtained. LCMS: MS (ESI) m/z 270.9.

### Step 3: Preparation of compound 1-4

Acetic acid (5.25 g, 87.43 mmol, 5 mL, and 57.06 eq) was added to ethanol (25 mL) solution of the compound 1-3 (500 mg, 1.53 mmol, and 1 eq) and the compound j (1.00 g, 4.23 mmol, and 2.76 eq), and it was reacted at room temperature (25 °C) for 16 hours. Reaction solution was concentrated under a reduced pressure, to obtain a compound k. LCMS: MS (ESI) m/z 489.1.

### Step 4: Preparation of compound 1

Cesium carbonate (3.45 g, 10.57 mmol, and 3.03 eq) was added to N,N-dimethylformamide (30 mL) solution of the compound k (1.9 g, 3.49 mmol, and 1 eq), the temperature was raised to 135 °C and it was reacted for 1 hour. Reaction solution (spread diatomaceous earth) was filtered, a filter cake was washed with N,N-dimethylformamide (30 mL), and a filtrate was concentrated under a reduced pressure. It was separated and purified by a column chromatography, to obtain a compound 1. LCMS: MS (ESI) m/z (M+H)⁺: 525.3.

### Step 5: Preparation of compound 1-4

Trifluoroacetic acid (6.16 g, 54.02 mmol, 4 mL, and 48.44 eq) was dropwise added to dichloromethane (16 mL) of the compound I (585 mg, 1.12 mmol, and 1 eq), and it was reacted at room temperature (30 °C) for 0.5 hours. Reaction solution was concentrated under a reduced pressure, to obtain a compound 1-4 (crude product, trifluoroacetate). LCMS: MS (ESI) m/z (M+1)⁺: 425.2.

### Step 6: Preparation of compounds 1A and 1B

Sodium carbonate (1.15 g, 10.85 mmol, and 4.30 eq) was added to tetrahydrofuran (10 mL) and water (10 mL) solution of the compound 1-4 (1.36 g, 2.53 mmol, 1 eq, and trifluoroacetate), tetrahydrofuran (1 mL) solution dissolved with acryloyl chloride (130 mg, 1.44 mmol, 117.12 µL, and 5.69 e-1 eq) was dropwise added to reaction solution, and it was reacted at room temperature (25 °C) for 1 hour. PH of the reaction solution was adjusted to about 5 by using 1 N hydrochloric acid, and it was extracted with dichloromethane (10 mL*3). An organic phase combined was dried with anhydrous sodium sulfate, and filtered, and a filtrate was concentrated under a reduced pressure. It was separated and purified by using a column chromatography, and after being detected by SFC (chromatographic column: Cellulose 2150×4.6 mml.D, 5 µm; mobile phase: A: supercritical carbon dioxide, B: 0.05% ethanol solution of diethylamine; gradient: B was increased from 5% to 40% within 5 min, and it was maintained at 40% for 2.5 min, and returned to 5% balance for 2.5 min; flow rate: 2.5 mL/min; column temperature: 35 °C; and wavelength: 220 nm), it was analyzed as a racemic compound. It was chirally separated (chromatographic column: Phenomenex-Cellulose-2 (250 mm*30 mm, 5µm); mobile phase: A: supercritical carbon dioxide, B: [0.1% NH₃H₂O ETOH]; B%: 40%-40%), to obtain a chiral wasomer compound 1A (the retention time was 6.616 min) and a compound 1B (the retention time was 6.971 min).

Compound 1A: ¹HNMR(400MHz, DMSO-*d*₆)δ9.09(s, 1H), 8.33(s, 1H), 7.85(d, J =9.0Hz, 2H), 7.39-7.22(m, 4H), 7.16-7.05(m, 1H), 6.92-6.76(m, 1H), 6.09(t, J = 16.1Hz, 1H), 5.72-5.57(m, 1H), 4.72(d, J=12.3Hz, 0.5H), 4.31(t, J=13.7Hz, 1H), 4.17-4.00(m, 0.5H), 3.53-3.44(m, 0.5H), 3.33-3.14(m, 1.5H), 3.12-3.02(m, 0.5H), 3.01-2.89(m, 0.5H), 2.27-2.16(m, 1H), 2.05-1.80(m, 2H), 1.66-1.46(m, 1H).LCMS:MS(ESI)m/z(M+1)⁺:479.2.

Compound 1B: ¹HNMR(400MHz, DMSO-*d*₆)δ9.09(s, 1H), 8.33(s, 1H), 7.85(d, J=8.8Hz, 2H), 7.40-7.23(m, 4H), 7.14-7.04(m, 1H), 6.94-6.75(m, 1H), 6.10(t, J = 16.1Hz, 1H), 5.72-5.52(m, 1H), 4.73(d, J=12.5Hz, 0.5H), 4.32(t, J=12.7Hz, 1H), 4.09(d, J=13.1Hz, 0.5H), 3.57-3.44(m, 0.5H), 3.33-3.15(m, 1.5H), 3.12-3.03(m, 0.5H), 3.03-2.88(m, 0.5H), 2.27-2.16(m, 1H), 2.05-1.79(m, 2H), 1.68-1.49(m, 1H). LCMS:MS(ESI)m/z(M+1)⁺:479.2.

### Embodiment 4: Research on hygroscopicity of crystal form A of compound in Formula (I)

### Experimental material:

SMS DVS Intrinsic Plus dynamic steam adsorption instrument.

### Experimental method:

A sample (10-20 mg) was taken and placed in a DVS sample tray for testing.

### Experimental result:

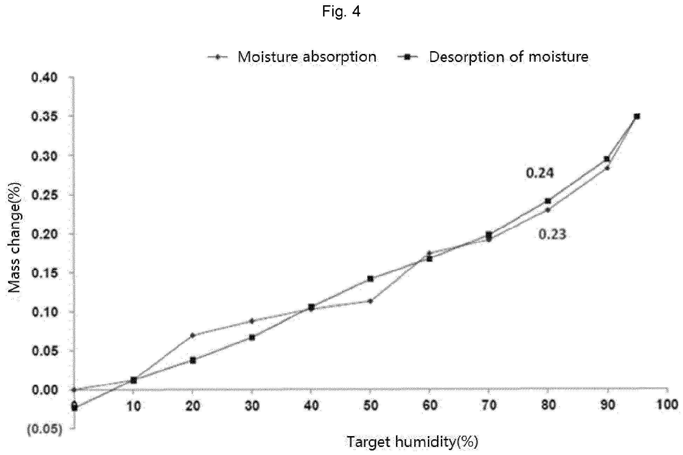

The DVS spectrum of the crystal form A of the compound in Formula (I) was shown in Fig. 4, and ΔW=0.23%.

### Experimental conclusion:

The crystal form A of the compound in Formula (I) showsed a hygroscopic weight increase of 0.23% at 80% RH/25°C, and the sample showed slight hygroscopicity.

### Embodiment 5: Solid stability test of crystal form A of compound in Formula (I)

According to the "Guiding Principles for Stability Test of Raw Materials and Preparations" (General Rule 9001 of Part Four of the Chinese Pharmacopoeia 2015 Edition), the stability of the crystal form A of the compound in Formula (I) was investigated under influencing factors ((high temperature (60 °C, open), high humidity (room temperature/relative humidity 92.5%, open), strong light (visible light intensity of 5000 lux and ultraviolet intensity of 90 w/cm², open), and accelerated conditions (40 °C/75% RH, open) and (60 °C/75% RH, open)).

About 20 mg of the crystal form A of the compound in Formula (I) was weighed respectively, and it was placed at the bottom of a glass sample bottle, and spread into a thin layer. Its sample was completely exposed for sampling. The samples placed under different conditions were sampled for testing (XRPD) on day 5, day 10, 1 month, 2 months, and 3 months. Test results were compared with an initial test result on day 0, and the test results were shown in Table 3 below:

**Table 3: Solid stability test results of crystal form A of compound A in Formula (I)**

| Test condition | Time point | Crystal form |
|---|---|---|
| - | Day 0 | Crystal form A |
| High temperature (60 °C, open) | Day 5 | Crystal form A |
| | Day 10 | Crystal form A |
| High humidity (25°C/relative humidity 92.5%, open) | Day 5 | Crystal form A |
| | Day 10 | Crystal form A |
| strong light (5000 Ix and ultraviolet intensity 90 w/cm², open) | Day 5 | Crystal form A |
| | Day 10 | Crystal form A |
| 40 °C/75%RH (open) | 1M | Crystal form A |
| | 2M | Crystal form A |
| | 3M | Crystal form A |
| 60 °C/75%RH (open) | 1M | Crystal form A |
| | 2M | Crystal form A |
| | 3M | Crystal form A |

Conclusion: the crystal form A of the compound in Formula (I) showed the good stability under both the influencing factors and accelerated conditions.

### Biological testing data:

Experimental example 1: EGFR, ITK, TEC, and BTK kinase test.

### 1. Reaction condition:

Buffer condition: 20 mM HEPES(pH 7.5), 10 mM MgCl₂, 1 mM EGTA, 0.02%Brij35, 0.02 mg/mL BSA, 0.1 mm Na₃VO₄, 2 mm DTT, 1 %DMSO.

### 2. Reaction procedure:

2.1. An indicator substrate was prepared in a reaction buffer prepared newly.

2.2. A cofactor required was transferred to the substrate solution above.

2.3. A kinase indicated was injected into the substrate solution and mixed gently.

2.4. An acoustic technology was used to convey a compound in DMSO to a kinase reaction mixture (Echo550).

2.5. ³³P-ATP (specific activity 0.01 µci/µL, finally) was fed into the reaction mixture to initiate a reaction. (ATP final concentrations were 2 µM, 5 µM, and 5 µM respectively).

2.6. The kinase reaction was incubated at room temperature for 120 min.

2.7. The reaction was recorded on P81 ion exchange paper (Whatman#3698-915).

2.8. 0.75% phosphoric acid was widely used to clean a filter.

2.9. The remaining radioactive phosphorylation substrate on filter paper was measured.

3. Data analysis: the kinase activity data was expressed as a percentage of the remaining kinase activity in the test sample compared to the carrier (dimethyl sulfoxide) reaction. The IC₅₀ value and curve fitting were obtained by using Prism4 software (GraphPad).

4. Experimental conclusion: results were shown in Table 4.

**Table 4: Comparison of EGFR, ITK, TEC and BTK kinase inhibitory activities**

| Compound number | EGFR (IC₅₀,nM) | ITK (IC₅₀,nM) | TEC (IC₅₀,nM) | BTK (IC₅₀,nM) | EGFR, ITK, TEC, and BTK activity ratio |
|---|---|---|---|---|---|
| 1B | 829 | 5430 | 74.8 | 2.16 | 383 times, 2513 times, 34 times |

Conclusion: the compound of the disclosure showed the better selectivity for EGFR, ITK, and TEC kinases.

## Claims

1. A crystal form A of a compound in Formula (I), wherein its X-ray powder diffractometer (XRPD) spectrum has characteristic diffraction peaks at the following 2θ angles: 17.7805±0.2000°, 22.0193±0.2000°, 27.4192±0.2000°,

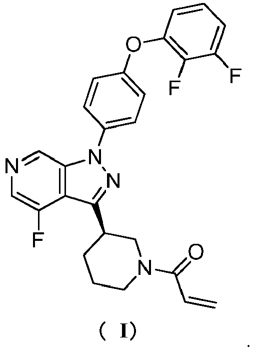

2. The crystal form A according to claim 1, wherein, its XRPD spectrum has characteristic diffraction peaks at the following 2θ angles: 15.0010±0.2000°, 17.0603±0.2000°, 17.7805±0.2000°, 22.0193±0.2000°, 23.5013±0.2000°, 27.4192±0.2000°.

3. The crystal form A according to claim 2, wherein, its XRPD spectrum has characteristic diffraction peaks at the following 2θ angles: 8.1387±0.2000°, 15.0010±0.2000°, 16.1591±0.2000°, 17.0603±0.2000°, 17.7805±0.2000°, 22.0193±0.2000°, 23.5013±0.2000°, 27.4192±0.2000°.

4. The crystal form A according to claim 3, wherein, its XRPD spectrum has characteristic diffraction peaks at the following 2θ angles: 8.1387±0.2000°, 13.6809±0.2000°, 15.0010±0.2000°, 16.1591±0.2000°, 17.0603±0.2000°, 17.7805±0.2000°, 22.0193±0.2000°, 23.5013±0.2000°, 24.0218±0.2000°, 27.4192±0.2000°.

5. The crystal form A according to claim 4, wherein, its XRPD spectrum has characteristic diffraction peaks at the following 2θ angles: 8.1387°, 9.8194°, 13.6809°, 15.0010°, 16.1591°, 17.0603°, 17.7805°, 18.4386°, 19.5400°, 21.3193°, 22.0193°, 23.1597°, 23.5013°, 24.0218°, 26.1791°, 26.6006°, 27.1199°, 27.4192°, 29.1595°, 29.7190°, 30.8417°, 31.2196°, 32.2992°, 32.9612°, 33.7773°, 34.3779°, 35.1796°, 37.1408°.

6. The crystal form A according to claim 5, wherein, its XRPD spectrum is basically shown in Fig. 1.

7. The crystal form A according to any one of claims 1-6, wherein, its differential scanning calorimeter (DSC) curve has a starting point of an endothermic peak at 107.89±3.00°C, and has a peak of an exothermic peak at 147.76±3.00°C.

8. The crystal form A according to claim 7, wherein, its DSC spectrum is shown in Fig. 2.

9. The crystal form A according to any one of claims 1-6, wherein, the weight loss of its thermal gravimetric analyzer (TGA) curve reaches 0.06% at 200.0±3 °C.

10. The crystal form A according to claim 9, wherein, its TGA spectrum is shown in Fig. 3.

11. The crystal form A according to claim 1, wherein, its single crystal X-ray diffraction data are as follows: monoclinic crystal system, space group C2, crystal cell parameters *a*=22.0128(6) Ǻ, *b*=12.7542(3) Ǻ, *c*=16.0152(4) Ǻ, α = γ = 90°, β = 90.4900(10)°, volume V=4496.2(2) Ǻ³, absolute configuration parameter Flack value of 0.04(3).

12. A preparation method for a crystal form A of a compound in Formula (I), comprising:
1) adding the compound in Formula (I) to a solvent to form solution;
2) adding an anti-solvent to the solution, stirring at a certain temperature for a period of time, and filtering, drying a filter cake under a reduced pressure;
wherein,
the solvent is an ester solvent, an ether solvent, or an alcohol solvent;
the anti-solvent is n-heptane, n-hexane, or water;
the stirring temperature is 0-40 °C;
the stirring time is 12-48 hours.

13. An application of the crystal form A according to any one of claims 1-11 in preparation of a drug for treating a disease related to BTK.

14. The application according to claim 13, wherein, the disease related to BTK is hematoma or autoimmune disease.
